# EUROPEAN PATENT SPECIFICATION

(11) **EP 3 522 855 B1**
(45) Date of publication and mention of the grant of the patent: **27.10.2021**
(21) Application number: 17780360.8
(22) Date of filing: 28.09.2017
(51) Int. Cl.: A61K 8/24, A61K 8/34, A61K 8/46, A61Q 11/00

(54) **PROCESS FOR MANUFACTURE OF NON-AQUEOUS ORAL CARE COMPOSITIONS**
VERFAHREN ZUR HERSTELLUNG WASSERFREIER ZUSAMMENSETZUNGEN FÜR DIE MUNDPFLEGE
PROCÉDÉ DE FABRICATION DE COMPOSITIONS NON-AQUEUSES POUR TRAITEMENT ORAL

(30) Priority: 10.10.2016 EP 16193162
(43) Date of publication of application: 14.08.2019
(73) Proprietor: Unilever IP Holdings B.V., 3013 AL Rotterdam (NL); Unilever Global IP Limited, Wirral, CH62 AZD (GB)
(72) Inventor: FORREST, Richard, Aaron, Wirral Merseyside CH63 3JW (GB); GROVES, Brian, Joseph, Wirral Merseyside CH63 3JW (GB); STARCK, Pierre, Wirral Merseyside CH63 3JW (GB)
(74) Representative: Tansley, Sally Elizabeth
(86) International application number: PCT/EP2017/074633
(87) International publication number: WO 2018/069054

(56) References cited:
- US-A- 5 254 334
- US-A- 5 409 706
- DATABASE WPI Week 198212 Thomson Scientific, London, GB; AN 1982-22872E XP002764860, & JP S57 26612 A (LION CORP) 12 February 1982 (1982-02-12) -& DATABASE EPODOC [Online] EUROPEAN PATENT OFFICE, THE HAGUE, NL; 12 February 1982 (1982-02-12), LION CORP: "PREPARATION OF ORAL COMPOSITION", XP002764737, Database accession no. JP-10177480-A -& JP S57 26612 A (LION CORP) 12 February 1982 (1982-02-12)

## Description

### Field of the Invention

The present invention is concerned with a process for preparing non-aqueous oral care compositions.

### Background of the Invention

Oral care compositions such as dentifrices typically contain dentally acceptable abrasive, humectant, water, and water-soluble polymer which serves as a thickener and binder for the ingredients. A variety of other ingredients such as flavours, sweeteners, preservatives and fluoride are also utilized at low levels.

However, the processes for preparing non-aqueous compositions incorporating the above ingredients can be difficult to conduct in a manner that produces a toothpaste with sensory properties that are acceptable to the consumer. Furthermore, many non-aquous compositions require structurants and process for incorporating these structurants can be difficult to perform in a manner that gives consistent rheological properties.

JP S57 26612 A (LION CORP) discloses a process for manufacturing an oral care non-aqueous composition comprising by the addition of an anionic surfactant to a polyol at a temperature such that the surfactant completely dissolves in the polyol followed by addition of sodium monofluorophosphate.

WO96/03108 describes a non-aqueous dentifrice composition comprising a carboxyvinyl polymer, a humectant, a polyethylene glycol and a dentally acceptable abrasive. The carboxyvinyl polymer is used to thicken the humectant materials and provide the necessary rheology in order to suspend any required abrasive material.

A further problem with non-aqueous formulations such as those disclosed in WO96/03108 is that they do not behave rheologically like a typical aqueous dentifrice.

This problem is observed both during manufacture and during use by the consumer. It has led to manufacturing difficulties and reduced acceptance amongst consumers.

The present invention relates to a process for producing non aqueous toothpastes with good sensory rheological parameters and without the need for further thickeners. Products made using these processing parameters are stable on storage.

### Summary of the Invention

The present invention provides a process for manufacturing a non-aqueous composition comprising the following steps:
i) addition of an anionic surfactant selected form the group consisting of, alfa olefin sulfonate, lauryl phosphate, sodium coco sulphate, sodium methyl cocoyl taurate, sodium methyl oleoyl taurate, sodium lauryl sulphate, sodium cocoyl isethionate, disodium lauryl sulfosuccinate, to a polyol at a temperature of 60°C or above such that the surfactant completely dissolves in the polyol followed by;
ii) addition of an alkali metal phosphate salt;
iii) cooling of the mix to a temperature below the solubility point of the surfactant in polyol, the cooling resulting in a structured mix,

in which the weight ratio of alkali metal phosphate salt to surfactant is from 1.1:1 to 10:1.

Use of surfactant crystals selected form the group consisting of, alfa olefin sulfonate, lauryl phosphate, sodium coco sulphate, sodium methyl cocoyl taurate, sodium methyl oleoyl taurate, sodium lauryl sulphate, sodium cocoyl isethionate, disodium lauryl sulfosuccinate, to structure a non-aqueous paste comprising polyol and alkali metal phosphate.

### Detailed Description of the Invention

The present invention relates to a process for manufacturing a non-aqueous composition comprising steps of adding an anionic surfactant to a polyol at a temperature such that the surfactant completely dissolves in the polyol. The surfactant is heated in the polyol to a temperature of 60°C or above.

Preferably if the composition comprises a perfume and/or flavouring, they are added after the mix is cooled to a temperature below the solubility point of the surfactant in the polyol. More preferably the perfume or flavouring is added to the mix after step iii) (cooling of the mix to a temperature below the solubility point of the surfactant in polyol) at a temperature of 45°C or below.

The composition of the invention is non-aqueous. By "non-aqueous" it is generally meant that water is not deliberately added to the composition in any significant quantity.

However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5%, more preferably no greater than about 3% more preferably no greater than 1 % by weight based on the total weight of the composition.

### Polyol

Compositions according to the invention comprises a polyol. Preferred polyols include organic polyols having 3 or more hydroxyl groups in the molecule (hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of polyol will depend on the particular type chosen, but generally ranges from about 20 to 90% by weight based on the total weight of the composition, the amount of organic polyol suitably ranges from 35 to 75%, more preferably from 45 to 70% by total weight organic polyol based on the total weight of the composition. Most preferably the composition of the invention is organic polyol-based. In the context of the present invention, the term "organic polyol-based" means that the composition is not oil-based or water-based, but instead, organic polyols (as defined above) are a principal component in the composition. By "principal component" is meant that the organic polyols (as defined above) when taken together, make up a higher portion of the composition's weight than any other compound. Ideally the composition of the invention is glycerol-based (i.e. glycerol makes up a higher portion of the composition's weight than any other compound) and contains from 45 to 70% by weight glycerol based on the total weight of the composition.

### Surfactant

Compositions of the invention comprise an anionic a surfactant.

The anionic surfactant is selected form the group consisting of, alfa olefin sulfonate, lauryl phosphate, sodium coco sulphate, sodium methyl cocoyl taurate, sodium methyl oleoyl taurate, sodium lauryl sulphate, sodium cocoyl isethionate, disodium lauryl sulfosuccinate and mixtures thereof. More preferably the anionic surfactant is selected from the group consisting of sodium coco sulphate, sodium methyl cocoyl taurate sodium lauryl sulphate (SLS) and mixtures thereof.

Preferably the level of anionic surfactant is from in an amount of from 0.2 to 5 wt%, more preferably from 1 to 3 wt% of the total composition.

### Alkali Metal Salt

Composition of the invention comprise an alkali metal phosphate salt. In a preferred embodiment, the phosphate salt used is one which results in an oral care composition (i.e., combined composition if a dual phase product) having a pH from 5.5 to 8, preferably from 6 to 7.5, and most preferably, about neutral.

Suitable phosphate salts include sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the phosphate salt used is trisodium phosphate and monosodium dihydrogen phosphate, more preferably at a trisodium phosphate to monosodium dihydrogen phosphate weight *ratio* of 1:4 to 4:1, most preferably, from 1:2 to 2:1, including all ratios subsumed therein.

The amount phosphate source(s) (e.g. trisodium phosphate and sodium dihydrogen phosphate) in the composition of this invention typically ranges from 1 to 15wt%, preferably from 2 to 10wt%, more preferably from 3 to 8% of the total composition.

Preferably the weight ratio of phosphate to surfactant is from 1.1:1 to 5:1.

### Thickening Agent

The liquid continuous phase of the composition of the invention is preferably free of thickening agent. In the context of the present invention this means that the compositions comprises less than 0.05 wt% of the total composition.

Such thickening agents include carboxyvinyl polymers.

### Product Form and Optional Ingredients

The composition of the invention is typically in the form of a dentifrice or serum. The term "dentifrice" denotes a formulation which are used to clean the surfaces of the oral cavity. The dentifrice is an oral composition that is not intentionally swallowed for purposes of systemic administration of therapeutic agents, but is retained in the oral cavity for a sufficient time to contact substantially all of the dental surfaces and/or mucosal tissues for purposes of oral activity. Preferably the dentifrice is suitable for application with a toothbrush and is rinsed off after use. Preferably the dentifrice is in the form of an extrudable semi-solid such as a cream, paste or gel (or mixture thereof).

A dentifrice composition according to the invention will generally contain further ingredients to enhance performance and/or consumer acceptability.

For example, the dentifrice may comprise other abrasive materials (in addition to the abrasive amorphous silica particles described above). Such other abrasive materials will generally be present in an amount of from 3 to 75% by weight based on the total weight of the dentifrice. Suitable other abrasive materials include other abrasive silicas (i.e. abrasive silicas which are different to the abrasive amorphous silica particles described above), calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Being non-aqueous, the composition of the invention is particularly suitable as a vehicle for oral care actives which may be physically or chemically incompatible with water, or which may function less efficiently in an aqueous environment.

Specific examples of oral care actives which may be included in the compositions of the invention include:
fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.

Preferred examples of oral care actives for inclusion in the compositions of the invention include agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably, the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Compositions of the present invention may also contain further optional ingredients customary in the art such as anticaries agents, anticalculus agents, buffers, flavouring agents, sweetening agents, colouring agents, opacifying agents, preservatives, antisensitivity agents, antimicrobial agents and the like.

Preferably the composition comprising a whitening agent. Suitable whitening agents comprise titanium dioxide, mica and blue dyes/pigments. Mixtures of whitening agents may be used.

### Process

A toothpaste was manufactured by the following steps:
i) Addition of an anionic surfactant to a polyol and heating to a temperature such that the surfactant completely dissolves in the polyol followed by;
ii) addition of other ingredients with the exception of alkali metal salt (phosphate), perfume and flavours;
iii) addition of alkali metal salt (phosphate) and allow time for solution to disperse;
iv) optionally;
v) cooling of the mix to a temperature below the solubility point of the surfactant in polyol;
vi) addition of perfumes and flavour;
vii) cooling resulting in a structured mix.

Constant Shear is present from the start of the process to the end. So the shear does not impact upon the invention but will help surfactants and metal salts to dissolve but their dissolution is mostly influence by temperature.

The invention is further illustrated with reference to the following, non-limiting Examples.

### EXAMPLES

Test formulations were prepared having the ingredients shown in Table 1 below.

All ingredients are expressed by weight percent of the total formulation, and as level of active ingredient, unless otherwise specified.

Structured formulation - Surfactant introduced early and dissolved before introduction of alkali metal salts are shown in Table 1:

**Table 1**

| **Ingredients** | **Example 1** | **Example 2** | **Example 3** |
|---|---|---|---|
| Glycerin | Balance | Balance | Balance |
| Sodium Lauryl sulfate | 2 | 2 | - |
| Sodium methyl cocoyl taurate | - | - | 2 |
| Calcium silicate (Ca₂SiO₃) | 10 | 15 | 15 |
| TiO2 | 15 | - | - |
| Sodium monofluorophosphate | 1.1 | 1.1 | 1.1 |
| Sweetener | 0.25 | 0.25 | 0.25 |
| Mica | - | 0.5 | 0.5 |
| Silica Abrasive | 6 | 6 | 6 |
| Monosodium dihydrogen phosphate | 1.5 | 3.5 | 3.5 |
| Trisodium phosphate | 1.5 | 3.5 | 3.5 |
| Calcium phosphate | 2 | 2 | 2 |
| Flavor | 1.1 | 1.1 | 1.1 |

The following process was used to prepare the Examples:
Stage 1:
   Add SLS, calcium silicate to cold glycerol or polyol and then heat from ambient to 60°C or 70°C - mix for 10 minutes at 60°C to 70oC.
Stage 2:
   Add SMFP, Sweetener and Titanium dioxide and mix for 10 minutes at 60oC to 70oC.
Stage 3:
   Add abrasives particles, calcium phosphate, monosodium phosphate, trisodium phosphate and mix for 20 minutes.
Stage 4:
   Cool to 45°C then add flavour, mix for 5 minutes and then cool to discharge the product.

Examples of Unstructured Formulation (eg surfactant introduced late or after the source of phosphate) are shown in Table 2.

**Table 2**

| **Ingredients** | **Example A** | **Example B** | **Example C** |
|---|---|---|---|
| Glycerin | Balance | Balance | Balance |
| Calcium silicate (Ca₂SᵢO₃) | 10 | 15 | 15 |
| Monosodium dihydrogen phosphate | 1.5 | 3.5 | 3.5 |
| Trisodium phosphate | 1.5 | 3.5 | 3.5 |
| TiO2 | 15 | - | - |
| Sodium monofluorophosphate | 1.1 | 1.1 | 1.1 |
| Sweetener | 0.25 | 0.25 | 0.25 |
| Mica | - | 0.5 | 0.5 |
| Silica Abrasive | 4 | 6 | 6 |
| Calcium phosphate | 2 | 2 | 2 |
| Sodium Lauryl sulfate | 2 | 2 | - |
| Sodium methyl cocoyl taurate | - | - | 2 |
| Flavor | 1.1 | 1.1 | 1.1 |

The commonly used process for the unstructured products:
Stage 1:
   Add calcium silicate, Sweetener, Titanium dioxide to cold glycerol and then heat from ambient to 60°C or 70°C - mix for 10 minutes at this temperature.
Stage 2:
   Add SMFP, Sweetener, monosodium phosphate, trisodium phosphate and mix for 10 minutes at 60oc to 70oC
Stage 3:
   Add titanium dioxide, abrasive and mix for 10 min.
Stage 4:
   Add calcium phosphate and surfactant and mix for 10 min.
Stage 5:
   Cool to 45°C then add flavour, mix for 5 minutes and then cool to discharge the product.

The viscosity (measured at 20°C) and yield stress of the Examples were measured and are shown in Table 3

**Table 3**

| | **Viscosity (Pa.s) at shear rate of 0.001 s⁻¹** | **Viscosity (Pa.s) at shear rate of 0.01 s⁻¹** | **Viscosity (Pa.s) at shear rate of 0.1 s⁻¹** | **Yield Stress (Pa)** |
|---|---|---|---|---|
| Example 1 SLS early | 10430 | 4771 | 935 | 110 Pa |
| Example A SLS late | 80 | 49 | 31 | No Yield Stress as unstructured |
| Example 2 SLS early | 13600 | 5982 | 1106 | 137 Pa |
| Example B SLS late | 125 | 120 | 78 | No Yield Stress as unstructured |
| Example 3 SMCT early | 550 | 381 | 200 | 5 Pa |
| Example C SMCT late | 95 | 70 | 48 | No Yield Stress unstructured |

Products made according to the invention (surfactant early and solubilised before introduction of alkali salts to polyol) do not need any extra structuring technologies (such as polymers) in them as these products hold their structure until a certain stress is applied to them - They typically exhibit a yield stress and behave as a toothpaste.

Product made with surfactant added late (which do not comprise structuring polymer) would flow under any stress and in fact would behave as a liquid or a thick slurry. They would therefore need extra structuring technology to achieve toothpaste properties.

## Claims

1. Process for manufacturing a non-aqueous composition comprising the following steps:
i) addition of an anionic surfactant selected form the group consisting of, alfa olefin sulfonate, lauryl phosphate, sodium coco sulphate, sodium methyl cocoyl taurate, sodium methyl oleoyl taurate, sodium lauryl sulphate,
sodium cocoyl isethionate, disodium lauryl sulfosuccinate, to a polyol at a temperature of 60°C or above such that the surfactant completely dissolves in the polyol followed by;
ii) addition of an alkali metal phosphate salt;
iii) cooling of the mix to a temperature below the solubility point of the surfactant in polyol, the cooling resulting in a structured mix,
in which the weight ratio of alkali metal phosphate salt to surfactant is from 1.1:1 to 10:1.

2. Process according to claim 1 in which a perfume and/or flavouring is added after the mix is cooled to a temperature below the solubility point of the surfactant in the polyol.

3. Process according to claim 1 or claim 2 in which the alkali metal salt is a sodium or potassium salt.

4. Process according to any preceding claim in which the phosphate is selected from monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate.

5. Process according to claim 4 in which the phosphate is selected from tri sodium phosphate and monosodium phosphate or mixtures thereof.

6. Process according to any preceding claim in which the total level of phosphate salt is from 2 to 10 wt% of the total composition.

7. Process according to any preceding claim in which the polyol is glycerol.

8. Process according to any preceding claim in which the anionic surfactant is selected from the group consisting of sodium coco sulphate, sodium methyl cocoyl taurate sodium lauryl sulphate (SLS) and mixtures thereof.

9. Process according to any preceding claim in which the weight ratio of phosphate to surfactant is from 1.1:1 to 5:1.

10. Process according to any preceding claim in which perfume or flavouring is added to the mix after step iii) of the process at 45°C or below.

11. Process according to any preceding claim in which the composition is a toothpaste.

12. Process according to any preceding claim in which further ingredients are added to the composition after the dissolution of the surfactant in the polyol (i).

13. Use of surfactant crystals selected from the group consisting of, alfa olefin sulfonate, lauryl phosphate, sodium coco sulphate, sodium methyl cocoyl taurate, sodium methyl oleoyl taurate, sodium lauryl sulphate, sodium cocoyl isethionate, disodium lauryl sulfosuccinate, to structure a non-aqueous tooth paste comprising polyol and alkali metal phosphate.

## Patentansprüche

1. Verfahren zur Herstellung einer nichtwässrigen Zusammensetzung, umfassend die folgenden Schritte:
i) Zugabe eines anionischen Tensids, ausgewählt aus der Gruppe bestehend aus alpha-Olefinsulfonat, Laurylphosphat, Natriumcocosulfat, Natriummethylcocoyltaurat, Natriummethyloleoyltaurat, Natriumlaurylsulfat, Natriumcocoylisethionat, Dinatriumlaurylsulfosuccinat, zu einem Polyol bei einer Temperatur von 60 °C oder darüber, so dass sich das Tensid vollständig in dem Polyol löst, gefolgt von;
ii) Zugabe eines Alkalimetallphosphatsalzes;
iii) Abkühlen der Mischung auf eine Temperatur unterhalb des Löslichkeitspunktes des Tensids in Polyol, wobei das Abkühlen zu einer strukturierten Mischung führt,
wobei das Gewichtsverhältnis von Alkalimetallphosphatsalz zu Tensid 1,1:1 bis 10:1 beträgt.

2. Verfahren nach Anspruch 1, bei dem ein Duftstoff und/oder Aromastoff zugegeben wird, nachdem die Mischung auf eine Temperatur unterhalb des Löslichkeitspunkts des Tensids in dem Polyol abgekühlt ist.

3. Verfahren nach Anspruch 1 oder Anspruch 2, bei dem das Alkalimetallsalz ein Natrium- oder Kaliumsalz ist.

4. Verfahren nach irgendeinem vorhergehenden Anspruch, bei dem das Phosphat ausgewählt ist aus Mononatriumphosphat, Natriumdihydrogenphosphat, Dinatriumhydrogenphosphat, Natriumpyrophosphat, Tetranatriumpyrophosphat, Natriumtripolyphosphat, Natriumhexametaphosphat, Kaliumdihydrogenphosphat, Trinatriumphosphat, Trikaliumphosphat.

5. Verfahren nach Anspruch 4, bei dem das Phosphat ausgewählt ist aus Trinatriumphosphat und Mononatriumphosphat oder Mischungen davon.

6. Verfahren nach irgendeinem vorhergehenden Anspruch, bei dem der Gesamtgehalt an Phosphatsalz 2 bis 10 Gew.-% der Gesamtzusammensetzung beträgt.

7. Verfahren nach irgendeinem vorhergehenden Anspruch, bei dem das Polyol Glycerin ist.

8. Verfahren nach irgendeinem vorhergehenden Anspruch, bei dem das anionische Tensid ausgewählt ist aus der Gruppe bestehend aus Natriumcocosulfat, Natriummethylcocoyltaurat, Natriumlaurylsulfat (SLS) und Mischungen davon.

9. Verfahren nach irgendeinem vorhergehenden Anspruch, bei dem das Gewichtsverhältnis von Phosphat zu Tensid 1,1:1 bis 5:1 beträgt.

10. Verfahren nach irgendeinem vorhergehenden Anspruch, bei dem der Mischung nach Schritt iii) des Verfahrens bei 45 °C oder darunter Duftstoff oder Aromastoff zugesetzt wird.

11. Verfahren nach irgendeinem vorhergehenden Anspruch, bei dem die Zusammensetzung eine Zahnpasta ist.

12. Verfahren nach irgendeinem vorhergehenden Anspruch, bei dem der Zusammensetzung nach dem Auflösen des Tensids in dem Polyol (i) weitere Inhaltsstoffe zugesetzt werden.

13. Verwendung von Tensidkristallen, ausgewählt aus der Gruppe bestehend aus alpha-Olefinsulfonat, Laurylphosphat, Natriumcocosulfat, Natriummethylcocoyltaurat, Natriummethyloleoyltaurat, Natriumlaurylsulfat, Natriumcocoylisethionat, Dinatriumlaurylsulfosuccinat, um eine nichtwässrige Zahnpasta zu strukturieren, die Polyol und Alkalimetallphosphat umfasst.

## Revendications

1. Procédé pour la fabrication d'une composition non aqueuse comprenant les étapes suivantes :
i) l'addition d'un tensioactif anionique choisi dans le groupe consistant en, sulfonate d'oléfine alpha, phosphate de lauryle, coco sulfate de sodium, méthyl cocoyl taurate de sodium, méthyl oléyl taurate de sodium, lauryl sulfate de sodium, cocoyl iséthionate de sodium, lauryl sulfosuccinate de disodium, à un polyol à une température de 60°C ou supérieure de sorte que le tensioactif se dissout complètement dans le polyol suivie par ;
ii) l'addition d'un sel de phosphate de métal alcalin ;
iii) le refroidissement du mélange à une température inférieure au point de solubilité du tensioactif dans le polyol, le refroidissement résultant en un mélange structuré,
dans lequel le rapport massique de sel de phosphate de métal alcalin à tensioactif est de 1,1:1 à 10:1.

2. Procédé selon la revendication 1, dans lequel un parfum et/ou arôme est ajouté après que le mélange est refroidi à une température inférieure au point de solubilité du tensioactif dans le polyol.

3. Procédé selon la revendication 1 ou revendication 2, dans lequel le sel de métal alcalin est un sel de sodium ou potassium.

4. Procédé selon l'une quelconque des revendications précédentes, dans lequel le phosphate est choisi parmi le phosphate de monosodium, dihydrogénophosphate de sodium, hydrogénophosphate de disodium, pyrophosphate de sodium, pyrophosphate de tétrasodium, tripolyphosphate de sodium, hexamétaphosphate de sodium, dihydrogénophosphate de potassium, phosphate de trisodium, phosphate de tripotassium.

5. Procédé selon la revendication 4, dans lequel le phosphate est choisi parmi le phosphate de trisodium et phosphate de monosodium ou des mélanges de ceux-ci.

6. Procédé selon l'une quelconque des revendications précédentes, dans lequel la teneur totale en sel de phosphate est de 2 à 10 % en masse de la composition totale.

7. Procédé selon l'une quelconque des revendications précédentes, dans lequel le polyol est le glycérol.

8. Procédé selon l'une quelconque des revendications précédentes, dans lequel le tensioactif anionique est choisi dans le groupe consistant en coco sulfate de sodium, méthyl cocoyl taurate de sodium, lauryl sulfate de sodium (SLS) et des mélanges de ceux-ci.

9. Procédé selon l'une quelconque des revendications précédentes, dans lequel le rapport massique de phosphate à tensioactif est de 1,1:1 à 5:1.

10. Procédé selon l'une quelconque des revendications précédentes, dans lequel le parfum ou arôme est ajouté au mélange après l'étape iii) du procédé à 45°C ou inférieur.

11. Procédé selon l'une quelconque des revendications précédentes, dans lequel la composition est un dentifrice.

12. Procédé selon l'une quelconque des revendications précédentes, dans lequel d'autres ingrédients sont ajoutés à la composition après la dissolution du tensioactif dans le polyol (i).

13. Utilisation de cristaux de tensioactif choisi dans le groupe consistant en, sulfonate d'oléfine alpha, phosphate de lauryle, coco sulfate de sodium, méthyl cocoyl taurate de sodium, méthyl oléoyl taurate de sodium, lauryl sulfate de sodium, cocoyl iséthionate de sodium, lauryl sulfosuccinate de disodium, pour structurer un dentifrice non aqueux comprenant du polyol et un phosphate de métal alcalin.
